# EUROPEAN PATENT APPLICATION

(11) **EP 1 050 314 A1**
(43) Date of publication of application: **08.11.2000**
(21) Application number: 00109151.1
(22) Date of filing: 08.05.2000
(51) Int. Cl.: A61L 27/02, A61L 27/10

(54) **Antimicrobial contact lens**

(30) Priority: 07.05.1999 US 310702
(71) Applicant: Healthshield Technologies L.L.C., West Hartford, CT 06106 (US)
(72) Inventor: Barry, John E., Derry, New Hampshire 03038 (US); Trogolo, Jeffrey A., Boston, Massachusetts 02116 (US)
(74) Representative: Wibbelmann, Jobst, Dr., Dipl.-Chem.

(57) **Abstract**

The present invention provides antimicrobial ocular lenses made from a polymeric material that contains an inorganic antimicrobial agent in order to make the lens resistant to microbial growth in the body or on the surface of the lens. In one embodiment the inorganic antimicrobial agent is a zeolite which contains antimicrobial metal ions. Lenses of the invention prevent microbial contamination and growth thereon by incorporating the inorganic antimicrobial agent into the lens material. A lens of the invention does not leach the antimicrobial agent into the eye during use or into ophthalmic solution in which it may be stored, and is safe (non-toxic) for *in vivo* use. Furthermore, substantial antimicrobial properties are not imparted to the solution, nor are the characteristics of ophthalmic solutions altered. As a result, all types of ophthalmic solutions can be used safely with a contact lens of the invention. Another advantage of the antimicrobial agent of the invention is that it does not cause substantial discoloration of the lens over time. In another aspect, the invention provides a method of killing or reducing microbes on a contact lens made from a polymeric material by adding an antimicrobial-effective amount of an inorganic antimicrobial agent to the polymeric material.

## Description

### FIELD OF THE INVENTION

The present invention relates to antimicrobial ocular lenses made from a polymeric material which contains an inorganic antimicrobial agent, the lens having antimicrobial surfaces, and the like.

### BACKGROUND OF THE INVENTION

Contact lenses are often exposed to microorganisms, which can cause infections in the eye after the infected lenses are worn. Conventional contact lenses should be periodically disinfected by the user to prevent infection or other deleterious effects on ocular health associated with contact lens wear. There are several conventional systems and methods aimed at preventing or at least reducing the microbial contamination of contact lenses, including sterilization solutions, enzymatic cleaners and heat sterilization cases.

Microbial contamination of the contact lens case which stores the lenses when not in use or during cleanings can also contribute to the problem of infection. The usual method employed to inhibit microbial growth on the case is to avoid its contamination to the extent possible, to cap the case, and to clean the case periodically. When a contact lens is placed in the case, it is usually immersed in ophthalmic solution which may provide a nutritive media for growth of bacteria and other microbes. However, antibacterial or preserved solutions are less effective against bacteria that are protected by a biofilm (a nutritive and protective film produced by microorganisms), which protects the bacteria by minimizing their contact with the solution.

Commercial ophthalmic solutions used for the care of contact lenses include saline solutions, lens cleaning solutions which incorporate a cleaning/disinfecting agent, and conditioning solutions. Since these solutions usually come into direct contact with the contact lens and the eye, many different preventative measures are taken to minimize contamination with microorganisms, such as infectious bacteria. These include avoiding bacterial contamination of the solution by packaging the solution under sterile conditions and designing the container to minimize the likelihood of contamination due to improper handling. Another method for preventing contamination is by incorporating a preservative into the solution that inhibits growth of microorganisms. In fact, many eye care systems designed to combat microbial growth are directed to making the ophthalmic solution antimicrobial.

Silver ions have been incorporated in ophthalmic solutions to prevent bacterial or microbial growth. For example, Schweisfurth et al., Contactologia, Vol 7D, pages 144-47 (1985) discloses antibacterial properties of silver-containing solutions used for storage and disinfection of contact lenses. Additionally, a sodium silver chloride complex (SoluSept, Similasan AG, Switzerland) has been used as a preservative for ophthalmic solutions. In each case, the silver imparts antimicrobial properties to the ophthalmic solution.

U.S. Pat. No. 5,320,843 issued to Raheja et al. provides a method for improving the antibacterial efficacy of an ophthalmic solution used in the care of contact lenses. The method uses a contact lens case which includes an aluminosilicate carrier, such as zeolite, retaining antibacterial metal ions. Ophthalmic solution is placed in contact with the article in order to render the solution antimicrobial; the zeolite releases metal ions into the ophthalmic solution in contact with the case.

Antimicrobial solutions containing silver ions can interact in unpredictable ways with certain components in the solution, and could inactivate the solution or adversely react with it. Such components include phosphate-buffered saline and NaCl. For example, the phosphate in PBS could precipitate with silver resulting in a change in pH of the ophthalmic solution. In addition, silver may react with chloride ions from NaCl to form silver chloride, which can cause serious irritation to the eye.

It would be highly advantageous instead to have a contact lens which has antimicrobial properties, in order to prevent the growth of microbes thereon and a consequential infection of the eye. A contact lens having antimicrobial properties has a reduced tendency to become contaminated with bacteria and other microbes, and can better tolerate the consequence of less stringent and less frequent disinfection procedures. Moreover, lenses need not be separately disinfected to decrease the possibility of microbial contamination. Antimicrobial lenses can be stored under conditions which usually would contribute to contamination of the lens, but due to its antimicrobial properties, the lenses remain in a disinfected or microbially uncontaminated state. Furthermore, a contaminated contact lens case is unlikely to spread microbes to the antimicrobial lenses, which are frequently stored in the case. In this manner, the chance or probability of contaminating an antimicrobial lens is reduced, and may even be substantially eliminated. Finally, since the ophthalmic solution does not contain the antimicrobial agent, formulators are not concerned with trying to balance the possible chemical reactions which can take place when adding an additional reactive component. Therefore, it is important to design an antimicrobial contact lens that does not release the antimicrobial agent into ophthalmic solution or the eye, to avoid adverse or undesired reactions.

U.S. Patent No. 5,340,583 issued to Dziabo et al. teaches a contact lens which is substantially silver-free and has a quaternary ammonium-containing organosilane as an antimicrobial component. The quaternary ammonium-containing organosilane molecules used in Dziabo et al. are large organic molecules, which may promote antibiotic resistance over time. Furthermore, these compounds can lead to irritation of the ocular tissues of sensitive users. Thus, it would be advantageous to provide a contact lens having an antimicrobial component, which is non-toxic and safe, without causing ocular irritation, and well tolerated in people.

### SUMMARY OF THE INVENTION

The present invention provides a means for preventing microbial contamination and growth on or in ocular lenses, by incorporating an inorganic antimicrobial agent into the lens material. A lens of the invention does not leach the antimicrobial agent into the eye during use or into ophthalmic solution in which it may be stored, and is safe (non-toxic) for *in vivo* use. Furthermore, substantial antimicrobial properties are not imparted to the solution, nor are the characteristics of ophthalmic solutions altered. As a result, all types of ophthalmic solutions can be used safely with a contact lens of the invention. Another advantage of the antimicrobial agent of the invention is that it does not cause substantial discoloration of the lens over time.

The antimicrobial characteristics of a lens of the invention reduce and potentially eliminate the likelihood of contamination, even without antimicrobial ophthalmic solution or conventional disinfecting techniques. If a conventional lens becomes contaminated, a biofilm may form on the surface of the lens or contaminated case as a result of colonization of contaminating microorganisms. The present invention provides a method of inhibiting the formation of such a biofilm on the surfaces of the contact lens without the use of an antimicrobial ophthalmic solution or conventional disinfecting means.

Thus, the present invention provides a novel antimicrobial ocular lens which is made from a polymeric material that contains an inorganic antimicrobial agent.

In another embodiment, the invention provides a contact lens made from a polymeric material which contains a silver zeolite, wherein the zeolite is between about 0.1 to 3 wt. % of the polymeric material and contains an amount of ion-exchanged silver ions in a concentration of about 0.01 to 5 wt. % of the zeolite and an amount of ion-exchanged ammonium ions in a concentration of about 0.5 to 15 wt % of the zeolite.

In another aspect, the invention provides a method of killing or reducing microbes on a contact lens made from a polymeric material by adding an antimicrobial-effective amount of an inorganic antimicrobial agent to the polymeric material in order to make the lens resistant to microbial growth in the body or on the surface of the lens.

### BRIEF DESCRIPTION OF THE DRAWING

**Fig. 1** is an elevation of a contact lens **11** having a lens body **12** in accordance with the present invention.
**Fig. 2** is a sectional elevation along line 2-2 of **Fig. 1** showing antimicrobial zeolite **13** dispersed throughout the lens body **12**.
**Fig. 3** is a diagramatic elevation of a contact lens **11** having antimicrobial zeolite **13** sprayed onto the surfaces of lens body **12**.

### DETAILED DESCRIPTION OF THE INVENTION

All patents, patent publications, and literature references cited in this specification are hereby incorporated by reference in their entirety.

As used herein, "antimicrobial" is synonymous with antibacterial, microbicidal, bactericidal, antibiotic, and the like, all of which may be used interchangeably, and means biocidal (*i.e.*, reducing substantially or eliminating the number of microbes on a surface) and/or merely inhibitory of microbial growth (also known as biostatic, *i.e.*, causing no increase in microbial growth) for a particular microbe, depending on the needs of the practitioner. The antimicrobial property of a substance can be assessed by testing under conditions in which the absence of the substance would permit microbes to grow.

As used herein, the term "antimicrobial agent" refers to an antimicrobial compound of the invention, or any portion thereof, having antimicrobial properties. For example, silver zeolite is an antimicrobial agent of the invention, and the antimicrobial silver ions that are retained by the zeolite are a "portion thereof" which are also an antimicrobial agent.

As used herein, the term "active", when used to describe an ingredient of a contact lens of the present invention, refers to the antimicrobial compounds or components described herein.

As used herein, the term "effective amount" is used synonymously with "sufficient amount", both terms referring to the amount of active ingredient in a lens of the invention required to achieve the antimicrobial properties thereof. This amount is at least equal to MBC (minimum biocidal content, *i.e.*, the content necessary to kill 99.9% of an inoculum of microbes within 24 hours of inoculation) when a biocidal effect is desired, and MIC (minimum inhibitory content) when an effect that is merely inhibitory of microbial growth is desired. The MIC and MBC are expressed as the minimum content of an antimicrobial agent in µg/cc. The smaller the value, the greater the efficacy of the antimicrobial agent.

As used herein "non-leachable" into ophthalmic solution in a substantially antimicrobial amount shall mean that the antimicrobial agent is not released from a lens which contains it, into a contacting aqueous media, including ophthalmic solution or the eye, over a period of twenty-four hours in an amount sufficient to impart to the media the antimicrobial property or characteristic that is desired of a lens of the invention. For example, the antimicrobial agent is substantially non-leachable in this manner if it imparts antimicrobial properties to a contact lens of the invention, but imparts no substantial antimicrobial activity to the contacting aqueous media after twenty-four hours of exposure as determined by the MIC or MBC (depending on the level of protection desired).

Although the present invention is described hereinafter in terms of a contact lens (including gas-permeable, soft, hard, etc.), other types of lenses, such as intraocular lenses, lenticules, corneal inlay and onlay lenses, and the like, can be made antimicrobial, thus having the properties described herein.

In its broadest aspect, the present invention is directed to a lens, preferably a corneal contact lens or simply a contact lens, structured to be used in a mammalian eye. The lens of the invention comprises a lens body, preferably an optically clear lens body, including a polymeric material and an antimicrobial-effective amount of an antimicrobial agent, preferably an inorganic antimicrobial agent which is substantially non-leachable under normal use conditions of the lens, and effective against at least one, preferably a plurality, and most preferably substantially all, of the microorganisms to which the lens is exposed, for example, while inside and/or outside of the eye.

The antimicrobial agent may be physically, chemically or otherwise combined with the polymeric material, either before or after the polymeric material is formed. The antimicrobial agent may be present primarily (*i.e.*, at least about 50%), or even substantially wholly, at or near the external surface of the lens body, *e.g.*, as a coating, or may be substantially uniformly distributed throughout the lens body.

In a preferred embodiment the inorganic antimicrobial agent is a metal ion retained on a ceramic carrier which is formulated in a contact lens of the invention to provide antimicrobial activity that is long lasting. Furthermore, such antimicrobial formulation used in the present invention kills microorganisms that contact the lens, including microorganisms which are exposed to a contact lens while in or out of the eye, over a prolonged period of time. Examples of such microorganisms include S*taphylococcus epidermidis, Staphylococcus aureous, Streptococcus pyogenes, Streptococcus pneumonia, Haemophilis influenzae*, and *Haemophilis aegyptius.*

In a particular embodiment of the lens of the invention, the antimicrobial agent is composed of a ceramic carrier such as zeolite, which retains an antimicrobial metal ion. Examples of other inorganic antimicrobial agents include hydroxy apatite, zirconium phosphates and various other ceramics that have been ion-exchanged with an antimicrobial metal ion (discussed *infra*). Hydroxy apatite particles containing antimicrobial metals are described in, e.g., U.S. Patent No. 5,009,898. Zirconium phosphates containing antimicrobial metals are described in, *e.g.*, U.S. Patent Nos. 5,296,238, 5,441,717 and 5,405,644. Other inorganic particles, such as the oxides of titanium, aluminum, zinc and copper, may be coated with a composition which confers antimicrobial properties, for example, by releasing antimicrobial metal ions such as silver ions, which are described, *e.g.*, in U.S. Patent No. 5,1890,585. Inorganic soluble glass particles containing antimicrobial metal ions, such as silver, are described, *e.g.*, in U.S. Patent Nos., 5,766,611 and 5,290,544. The following patent publications disclose antimicrobial zeolite and various polymer articles having antibacterial properties which contain the zeolite: U.S. Pat. No. 4,525,410; U.S. Pat. No. 4,775,585; U.S. Pat. No. 4,906,464; U.S. Pat. No. 4,911,898; U.S. Pat. No. 4,911,899; U.S. Pat. No. 4,938,955; U.S. Pat. No. 4,938,958; U.S. Pat. No. 5,003,638; U.S. Pat. No. 5,064,599; U.S. Pat. No. 5,085,416; Japanese Patent Abstract Publication No. 02080442; and Japanese Patent Abstract Publication No. 02125717.

"Zeolite" is an aluminosilicate having a three dimensional skeletal structure that is represented by the formula: XM_{2/*n*}O-Al₂O₃-YSiO₂-ZH₂O. M represents an ion-exchangeable ion, generally a monovalent or divalent metal ion, *n* represents the atomic valency of the (metal) ion, X and Y represent coefficients of metal oxide and silica respectively, and Z represents the number of water of crystallization. Examples of such zeolites include A-type zeolites, X-type zeolites, Y-type zeolites, T-type zeolites, high-silica zeolites, sodalite, mordenite, analcite, clinoptilolite, chabazite and erionite. The present invention is not restricted to any specific types of zeolite carriers. Natural zeolites and synthetic zeolites can be used to make the antimicrobial zeolites of the present invention.

Antimicrobial zeolite for use in a lens of the invention is prepared according to conventional methods known in the art, which methods are described in the patents listed *supra*. Zeolite particles preferably have a particle diameter size from about 0.2 to 40 µm, more preferably between about 0.5 to 5 µm. They include the antimicrobial zeolites disclosed, for example, in U.S. Patent Nos. 4,938,955 issued to Niira et al. and 4,911,898 issued to Hagiwara et al. The zeolite of Niira '955 is preferred because it contains ion-enhanced ammonium which prevents metal ions retained thereon from quickly leaching in large quantities into an aqueous media in contact therewith, *e.g.*, ophthalmic solution or the eye. Zeolite retaining metal ions that is prepared in accordance with Niira '955 is an example of one formulation of zeolite that can be used advantageously to form an antimicrobial contact lens of the invention.

The specific surface area of preferred zeolite particles is at least about 150 m²/g (anhydrous zeolite as standard) and the SiO₂/Al₂O₃ mol ratio in the zeolite composition is preferably less than 14, more preferably less than 11.

The antimicrobial metal ions can be retained on the zeolite particles through an ion-exchange reaction. Antimicrobial metal ions which are merely adsorbed or attached without an ion-exchange reaction exhibit a decreased bactericidal effect and their antimicrobial properties are not as long-lasting.

The ion-exchange capacities of these zeolites are as follows: A-type zeolite = 7 meq/g; X-type zeolite = 6.4 meq/g; Y-type zeolite = 5 meq/g; T-type zeolite = 3.4 meq/g; sodalite = 11.5 meq/g; mordenite = 2.6 meq/g; analcite = 5 meq/g; clinoptilolite = 2.6 meq/g; chabazite = 5 meq/g; and erionite = 3.8 meq/g. These ion-exchange capacities are sufficient for the zeolites to undergo ion-exchange with ammonium and antimicrobial metal ions.

In the ion-exchange process, the antimicrobial metal ions (cations) tend to be converted into their oxides, hydroxides, basic salts, etc., either in the micropores or on the surfaces of the zeolite and also tend to deposit there, particularly when the concentration of metal ions in the vicinity of the zeolite surface is high. Such deposition can adversely affect the bactericidal properties of ion-exchanged zeolite. In usual methods of ion-exchanging the sodium ions of the zeolite for other metal ions, a rather high concentration of the metal ions is used to obtain a high degree of ion exchange. In the zeolites preferred in the present invention, a relatively low degree of ion exchange is not only satisfactory, but also essential for better bactericidal properties of ion-exchanged zeolite. The preferred zeolite retains the bactericidal metal ions in an amount up to about 41% of the theoretical ion-exchange capacity of the zeolite.

In antimicrobial zeolite particles used in the present invention, ion-exchangeable ions present in zeolite, such as sodium ions, calcium ions, potassium ions and iron ions are partially replaced with ammonium and antimicrobial metal ions. Such ion-exchangeable ions may co-exist in the antimicrobial zeolite particle since they do not hinder the antimicrobial effect.

The preferred antimicrobial metal ion is silver. Antimicrobial metal ions (cations) other than silver can be ion-exchanged in the zeolite, including cations of gold, copper, zinc, mercury, tin, lead, bismuth, cadmium, chromium and thallium. Non-toxic antimicrobial ions include silver, gold, copper, and zinc ions.

Antimicrobial metal ions, such as silver ions, are believed to exert their antimicrobial effects by disrupting respiration and electron transport systems upon absorption into bacterial or fungal cells. Antimicrobial silver ions are particularly safe due to the fact that they are not substantially absorbed into the body. Thus, use of silver containing compositions allows for an exceptionally safe, non-toxic formulation that is useful in contact lenses. Thus, the invention provides a means for preventing growth of microbes by killing organisms that would otherwise contaminate a contact lens lacking the antimicrobial properties of the invention. More preferably, the invention provides a means for killing or preventing the growth of bacteria in or on a lens, which bacteria include, but are not necessarily limited to *S. aureus*, *S. epidermidis*, *C*. *albicans*, *P. vulgaris*, *S. mutans*, and *P. aeruginosa*.

The antimicrobial component of the lens does not migrate into or through a liquid medium to provide the desired antimicrobial properties. Instead, the antimicrobial components are substantially non-leachable from the lens into the liquid media. Furthermore, in a preferred embodiment using zeolite containing ion-exchanged ammonium ions (in addition to ion-exchanged silver) wherein the ammonium ions are present in a concentration of 0.5 to 15% by weight of the zeolite, no substantial "spike" effect is observed. Therefore, significant amounts of silver ion are not released when the zeolite in the lens is contacted with aqueous solution. Thus, the antimicrobial components of the invention remain associated with the lens, do not contaminate fluids in the eye or liquids used to care for contact lenses and are effective for relatively long periods of time, even indefinitely.

Ion release rate experiments show that an antimicrobial agent of the invention can effect antimicrobial action to a lens over long periods of time without substantial leaching of antimicrobial particles into a solution in contact with the lens. The release rate of antimicrobial metal ions from zeolite in a typical lens of the invention is less than about 100 parts per billion (ppb) per day, which release rate is substantially constant over time, *i.e*., there is no "spike" effect. Therefore, the lens does not make a solution in contact therewith antimicrobial, or release amounts of metal ions of any significance into the eye, which may be of concern to the public despite the fact that certain antimicrobial metal ions are nontoxic, *e.g.*, silver.

The antimicrobial metal ion is present in a concentration from about 0.01 to 5 wt. % of the zeolite to impart the desired antimicrobial properties to a lens of the invention. The antimicrobial particles are preferably present in a concentration of about 0.01 to 5% by weight of the polymeric material. The concentration will vary depending on the amount of silver in the zeolite. Antimicrobial zeolite is preferably present in an amount from about 0.1 to 3% by weight of the polymeric material to prevent substantial discoloration of the lens.

A preferred antimicrobial zeolite for use in a lens of the invention is Type A zeolite containing either a combination of ion-exchanged silver, zinc, and ammonium, or silver and ammonium. One such zeolite is manufactured by Shinagawa, Inc. (Japan) under the product number AW-10N, and consists of 0.6% by weight of silver ion-exchanged in Type A zeolite particles having a diameter of about 2.5µ. Another formulation, AJ-10N, consists of about 2% by weight silver ion-exchanged in Type A zeolite particles having a diameter of about 2.5µ. Another formulation, AW-80, contains 0.6% by weight of silver ion-exchanged in Type A zeolite particles having a diameter of about 1.0µ. Another formulation, AJ-80N, consists of about 2% by weight silver ion-exchanged in Type A zeolite particles having a diameter of about 1.0µ. These zeolites contain from about 0.5% to 14% by weight of ion-exchanged ammonium. The zeolites are often obtained in master batches of low density polyethylene, polypropylene, or polystyrene, containing 20 weight % of the zeolite.

The antimicrobial properties of aqueous formulations of the inorganic antimicrobial agents of the invention may be assayed using conventional techniques, including for example determining the MIC or MBC with respect to a variety of bacteria, eumycetes, yeast, and other microbes. Assays for determining the MIC or MBC are well known in the art. Briefly, a mixture containing a particular microorganism is smeared onto a culture plate containing culture medium to which a test sample of antimicrobial compound of the invention is added at varying content or concentrations. The inoculated plate is incubated and cultured to determine the presence of growth of the microorganism. In these tests the following microorganisms, in addition to those listed above, may be employed: *Streptococcus mutans, Porphyromonas gingivalis, Bacillus cereus var mycoides, Escherichia coli, Pseudomonas aeruginosa, Staphylococcus aureus, Streptococcus faecalis, Aspergillus niger, Aureobasiduim pullulans, Chaetomium globosum, Gliocladium virens, Penicillum funiculosum, Candida albicans*, and *Saccharomyces cerevisiae.*

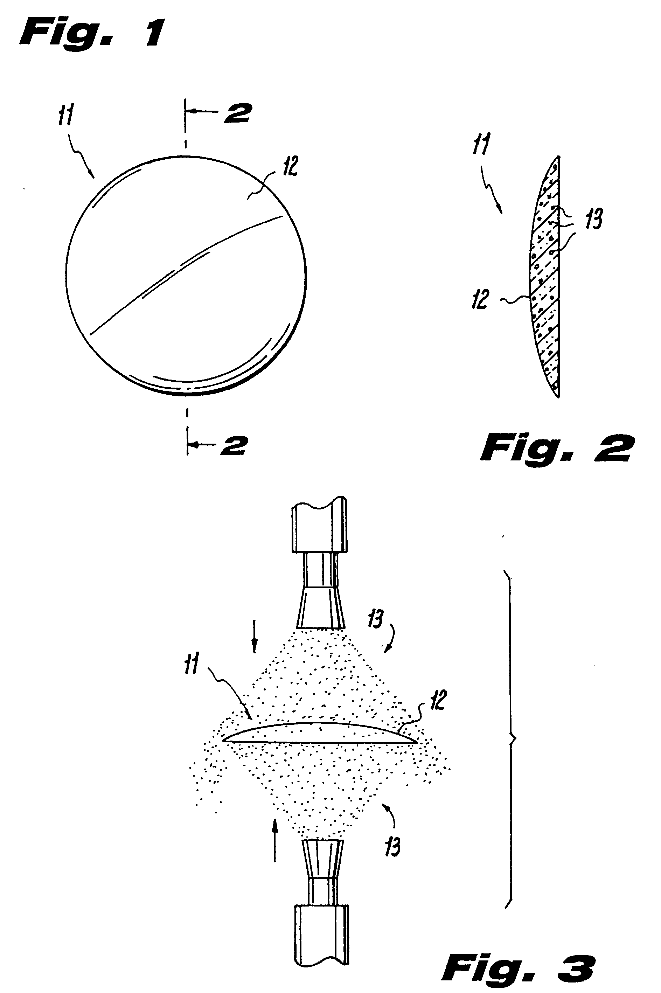

Referring now to the drawing, **Fig. 1** and **Fig. 2** illustrate a contact lens **11** in accordance with the present invention. Contact lens **11** includes an optically clear contact lens body **12** and is useful as a conventional contact lens on the cornea of a mammalian eye to correct the vision.

Contact lens body **12** is made of a material which includes a suitable polymeric material and an effective inorganic antimicrobial agent, such as in this particular embodiment, silver zeolite **13**. The antimicrobial agent is present in an amount effective against at least one, preferably a plurality, and even substantially all of the microorganisms to which contact lens **11** is exposed. Antimicrobial zeolite **13** in contact lens **11** is ophthalmically acceptable. That is, the antimicrobial component of the contact lens is non-toxic, does not have, or cause, significant adverse effects on the wearer, in particular on the ocular health of the wearer, and does not cause substantial discoloration of the lens.

In formulating the material for use in contact lens body **12**, the material should be optically clear, suitable for use in the eye, and otherwise suitable as a material for construction of a contact lens. The polymeric material useful in contact lens **11** may be substantially any such material useful as a component in corneal contact lenses, for example, conventional corneal contact lenses. This polymeric material may comprise polymers that are synthetic, naturally occurring, or a combination thereof. Examples of polymeric materials that can be used in contact lens body **12** include silicone polymers, such as polysiloxanes and the like; polyolefins, such as polyethylene, polypropylene, and the like; polyesters; polyurethanes; acrylic polymers, such as polyacrylates and polymethacrylates; hydrogel-forming polymers; polycarbonates; and combinations thereof. Specific examples of polymeric materials include polyhydroxyethyl methacrylate (poly-HEMA), polyacrylimide, polydimethyl siloxane, polyvinyl-2-pyrrolidinone, silicone-acrylate, or other hydrophilic contact lens material, and the like. The polymer or copolymer should be optically clear and otherwise useful as a contact lens material.

Contact lens **11** can include additional components, such as UV light absorbers, dyes and the like, which are commonly present in contact lenses in an amount effective to provide one or more additional desired properties to the lens.

Contact lens **11** can be provided with the inorganic microbial agent in any suitable manner. In one embodiment, a pre-formed contact lens (*i.e.*, the lens is already formed), such as contact lens **11**, is contacted with an inorganic antimicrobial agent of the invention under conditions effective to retain the agent on the lens. In this embodiment, the antimicrobial component is preferably present primarily, and even substantially wholly, at or near an external surface, for example, all the external surfaces, of the body **12** of the contact lens.

The antimicrobial contact lens can be prepared by applying a coating solution containing an inorganic antimicrobial agent onto the lens by, for example, direct spray coating or dip coating, *i.e.*, the antimicrobial solution is applied by spraying the contact lens with antimicrobial solution as depicted in **Fig. 3** or by dipping the contact lens into the coating solution, respectively. These coating and spraying processes are known generally in the art.

The coating material includes polymers, *e.g.*, hydrophilic polymers and copolymers as well as hydrogels, or any other suitable polymer that can be coated to the surface of a lens of the invention. The concentration of inorganic antimicrobial agent used in the coating solution is between 0.01 and 20%, preferably between 0.02 and 10%, and most preferably between 0.05 and 5%.

In another embodiment, a contact lens body, such as contact lens body **12**, is prepared by polymerizing one or more monomers or copolymers chosen to provide the polymeric material and the antimicrobial component. The inorganic antimicrobial zeolite must be incorporated into the polymer while it is in the precursor form before polymerization. To do this, a powder containing the antimicrobial agent, *e.g.*, zeolite powder, is dispersed in the precursor to produce concentrated slurry using a high shear mixer to ensure that little or no agglomeration occurs. The slurry concentrate is then added to the precursor while mixing to result in the concentration desired. The slurry concentrate is then reacted (crosslinked) with the polymer to from the final polymer. Crosslinking can be induced chemically, through heating or by radiation such as UV.

For cast polymers using a solvent process, the inorganic antimicrobial agent can be added to the polymer-solvent mix by first dispersing in the solvent alone, and then mixing that combination with the polymer-solvent. In this manner, the resulting polymer or copolymer has a substantially nonleachable, covalently bonded inorganic antimicrobial agent present in the polymer structure.

The antimicrobial component is preferably substantially uniformly distributed throughout the polymer or copolymer. The polymer or copolymer can then be formed into a contact lens, such as contact lens **11**, using conventional means such as molding or machining.

Once contact lens **11** is formed, it has sufficient antimicrobial activity to at least prolong the time between separate disinfections of the lens. This feature at least reduces the need to separately disinfect contact lens **11** and, therefore, reduces the amount of care that a contact lens wearer needs to take without detrimentally affecting or risking ocular health. Preferably, the antimicrobial contact lens **11** has sufficient antimicrobial activity so that no separate disinfection procedure of the lens is required. In this embodiment, contact lens **11** can be stored in a commercially available preserved contact lens soaking solution while the lens is not in use in the eye. Such soaking is satisfactory to maintain contact lens **11** suitably free of microorganisms without the need for a separate disinfection procedure, for example, contacting contact lens **11** with hydrogen peroxide.

## Claims

1. An ocular lens comprising a polymeric material wherein the polymeric material comprises an antimicrobial-effective amount of an antimicrobial zeolite, the lens being resistant to microbial growth within the body or the surface thereof.

2. The lens of claim 1 wherein the amount of antimicrobial zeolite is about 0.01 to 5 wt. % of the polymeric material.

3. The lens of claim 2 wherein the antimicrobial zeolite comprises ion-exchanged antimicrobial metal cations in a concentration of about 0.01 to 5 wt. % of the zeolite.

4. The lens of claim 3 wherein the zeolite comprises ion-exchanged ammonium ions in a concentration of about 0.5 to 15 wt % of the zeolite.

5. The lens of claim 1 wherein the polymeric material is selected from silicone polymers, polyolefins, polyesters, polyurethanes, acrylic polymers, hydrogel-forming polymers, and polycarbonates.

6. The lens of claim 1 wherein the inorganic antimicrobial agent is dispersed in the polymeric material.

7. The lens of claim 1 having a surface coating comprising the polymeric material and the inorganic antimicrobial agent.

8. The lens of claim 1 wherein the lens does not leach the inorganic antimicrobial agent from the polymeric material into an aqueous medium in contact with the lens in an amount greater than 100 ppb following twenty-four hour exposure.

9. The lens of claim 1 which is a contact lens or an intraocular lens.

10. A contact lens comprising a polymeric material wherein the polymeric material comprises an antimicrobial-effective amount of a silver zeolite.

11. The contact lens of claim 10 wherein the polymeric material comprises the silver zeolite in a concentration of about 0.01 to 5 wt. % of the polymeric material.

12. The lens of claim 10 wherein the silver zeolite comprises ion-exchanged silver ions in a concentration of about 0.01 to 5 wt. % of the zeolite.

13. The lens of claim 10 wherein the silver zeolite comprises ion-exchanged ammonium ions in a concentration of about 0.5 to 15 wt % of the zeolite.

14. The lens of claim 10 wherein the polymeric material is selected from silicone polymers, polyolefins, polyesters, polyurethanes, acrylic polymers, hydrogel-forming polymers, and polycarbonates.

15. The lens of claim 10 wherein the silver zeolite is dispersed in the polymeric material.

16. The lens of claim 10 having a surface coating comprising the polymeric material and the silver zeolite.

17. A contact lens comprising a polymeric material which comprises a silver zeolite, wherein the silver zeolite is present in the polymeric material at a concentration of about 0.1 to 3 wt. % of the polymeric material and comprises an amount of ion-exchanged silver ions in a concentration of about 0.01 to 5 wt. % of the zeolite and an amount of ion-exchanged ammonium ions in a concentration of about 0.5 to 15 wt % of the zeolite.

18. The lens of claim 17 wherein the polymeric material is selected from silicone polymers, polyolefins, polyesters, polyurethanes, acrylic polymers, hydrogel-forming polymers, and polycarbonates.

19. The lens of claim 17 wherein the silver zeolite is dispersed in the polymeric material.

20. The lens of claim 17 comprising a surface coating comprised of the polymeric material and the silver zeolite.

21. The lens of claim 17 wherein the lens does not leach the silver zeolite from the polymeric material into an aqueous medium in contact with the lens in an amount greater than 100 ppb following twenty-four hour exposure.

22. A method of killing or reducing microbes on a contact lens comprised of a polymeric material, comprising adding an antimicrobial-effective amount of an inorganic antimicrobial agent to the polymeric material.

23. The method of claim 22 wherein the added antimicrobial agent is substantially present at or near an external surface of the contact lens.

24. The method of claim 22 wherein the added antimicrobial agent is dispersed throughout the polymeric material.

25. The method of claim 22 wherein the antimicrobial agent comprises an antimicrobial metal cations retained on a zeolite.

26. The method of claim 25 wherein the antimicrobial agent is silver zeolite.

27. The method of claim 22 wherein the microbe is selected from *S. aureus, S. epidermidis, C. albicans, P. vulgaris, S. mutans* and *P. aeruginosa.*

28. An ocular lens comprising a polymeric material wherein the polymeric material comprises an antimicrobial-effective amount of an inorganic antimicrobial agent.
